(19) Europäisches Patentamt
European Patent Office
Office européen des brevets

(11) EP 4 297 719 B1

(12) EUROPEAN PATENT SPECIFICATION

(45) Date of publication and mention of the grant of the patent:
**19.02.2025 Bulletin 2025/08**

(21) Application number: **22709128.7**

(22) Date of filing: **22.02.2022**

(51) International Patent Classification (IPC):
***A61K 8/73*** *(2006.01)* ***A61Q 5/02*** *(2006.01)*
***A61Q 19/10*** *(2006.01)*

(52) Cooperative Patent Classification (CPC):
**A61K 8/731; A61Q 5/02; A61Q 19/10;**
A61K 2800/48

(86) International application number:
**PCT/US2022/017188**

(87) International publication number:
**WO 2022/182616 (01.09.2022 Gazette 2022/35)**

(54) **PERSONAL CARE RINSE OFF COMPOSITION COMPRISING CROSSLINKED CELLULOSE ETHER**

KÖRPERPFLEGE-SPÜLZUSAMMENSETZUNG MIT VERNETZTEM CELLULOSEETHER

COMPOSITION DE RINÇAGE POUR SOINS PERSONNELS COMPRENANT DE L'ÉTHER DE CELLULOSE RÉTICULÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.02.2021 US 202163153403 P**

(43) Date of publication of application:
**03.01.2024 Bulletin 2024/01**

(73) Proprietors:
- **Dow Global Technologies LLC**
  **Midland, MI 48674 (US)**
- **Dow Silicones Corporation**
  **Midland, MI 48686-0994 (US)**
- **Rohm and Haas Company**
  **Collegeville, PA 19426 (US)**

(72) Inventors:
- **PARADA, German**
  **Midland, Michigan 48686 (US)**
- **LEAL, Lyndsay M.**
  **Collegeville, Pennsylvania 19426 (US)**
- **HILD, Alexandra**
  **29699 Walsrode (DE)**
- **NEUBAUER, Jörg**
  **29699 Walsrode (DE)**
- **JOHNSON, Bethany K.**
  **Midland, Michigan 48686 (US)**

(74) Representative: **Jewell, Catherine Mary**
**Beck Greener LLP**
**Fulwood House**
**12 Fulwood Place**
**London WC1V 6HR (GB)**

(56) References cited:
**WO-A1-2017/004119**
**US-A1- 2019 274 944**
**US-A1- 2020 155 439**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The present invention relates to an aqueous personal care rinse off composition. In particular, the present invention relates to an aqueous personal care rinse off composition, comprising: a dermatologically acceptable aqueous vehicle; a dermatologically acceptable cleaning surfactant; and a suds enhancing structurant; wherein the suds enhancing structurant comprises a crosslinked cellulose ether containing 0.1 to 1.0 wt%, based on weight of the crosslinked cellulose ether, of polyether groups.

Effective thickeners for use in personal care applications that also provide additional benefits to the formulator are in continuing demand. Of particular interest is an effective thickener that is non-ionic so as to be compatible in conventional personal care formulations, particularly since many such formulations typically include crosslinked and neutralized polyacrylic acid.

Nonionic cellulose ethers have been known to be used as thickening agents in aqueous paint compositions, for instance in latex containing paint compositions for providing stability, consistency and water retention to the aqueous paint compositions. Furthermore, nonionic cellulose ethers readily combine with frequently occurring ingredients in the paint compositions.

One nonionic cellulose ether often used in aqueous paint compositions is hydroxethyl cellulose ethers which lack a flocculation temperature below 100 °C in water. Such cellulose ethers are desirable since they normally, when used in aqueous paint compositions, contribute to a stable viscosity, a low tendency to flocculate organic and inorganic pigments and have a low tendency to form stable foams. However, the conventional hydroxyethyl cellulose ethers have the disadvantage of a comparatively weak thickening effect and little or no wetting ability.

Accordingly, there is a need for a thickener that is not only nonionic and that offers effective thickening for a variety of personal care formulation areas, but also one that is naturally derived for improved biodegradability and sustainability.

One improved biodegradable/sustainable thickener is disclosed by Adamo et al in U.S. Patent No. 8,999,391. Adamo et al. teach a personal care formulation comprising: methyl ethyl hydroxyethyl cellulose having a methyl DS to ethyl DS ratio of from about 1 to about 6.5; and at least one cosmetically acceptable ingredient, wherein the weight average molecular weight of the methyl ethyl hydroxyethyl cellulose is from about 100,000 to about 3,000,000 Daltons. US 2020/155439 relates to a personal care composition containing: a vehicle; a surfactant; a water-soluble cellulose ether base material substituted with a hydrophobic group having a carbon chain with 8 to 15 carbon atoms.

Notwithstanding, conventional cellulose ethers thickeners frequently impart an undesired stringy consistency to formulations. Accordingly, there remains a need for structurants for use in aqueous personal care rinse off compositions; particularly sustainable structurants that provide a luxuriant feel to the composition while not giving the composition a stringy consistency.

The present invention provides an aqueous personal care rinse off composition, comprising: a dermatologically acceptable aqueous vehicle; a dermatologically acceptable cleaning surfactant; and a suds enhancing structurant; wherein the suds enhancing structurant comprises a crosslinked cellulose ether containing 0.1 to 1.0 wt%, based on weight of the crosslinked cellulose ether, of polyether groups.

The present invention provides an aqueous personal care rinse off composition, comprising: a dermatologically acceptable aqueous vehicle; a dermatologically acceptable cleaning surfactant; and a suds enhancing structurant; wherein the suds enhancing structurant comprises a crosslinked cellulose ether containing 0.1 to 1.0 wt%, based on weight of the crosslinked cellulose ether, of polyether groups; wherein the aqueous personal care rinse off composition is selected from the group consisting of a shampoo, a conditioning shampoo, a body wash formulation, an exfoliating body wash formulation, a facial wash formulation, an exfoliating facial wash formulation, a liquid hand soap formulation, a sulfate-free cleansing formulation and a mild cleansing formulation.

The present invention provides an aqueous personal care rinse off composition, comprising: a dermatologically acceptable aqueous vehicle; a dermatologically acceptable cleaning surfactant; and a suds enhancing structurant; wherein the suds enhancing structurant comprises a crosslinked cellulose ether containing 0.1 to 1.0 wt%, based on weight of the crosslinked cellulose ether, of polyether groups; wherein the aqueous personal care rinse off composition is selected from the group consisting of a shampoo, a conditioning shampoo, a body wash formulation, an exfoliating body wash formulation, a facial wash formulation, an exfoliating facial wash formulation, a liquid hand soap formulation, a sulfate-free cleansing formulation and a mild cleansing formulation; and wherein the crosslinked cellulose ether is an irreversibly crosslinked cellulose ether.

The present invention provides a method of cleaning at least one of mammalian skin and hair, comprising: (a) applying an aqueous personal care rinse off composition of the present invention to the skin or hair of a mammal; and (b) rinsing the aqueous personal care rinse off composition from the skin or hair with a rinse water.

## DETAILED DESCRIPTION

We have surprisingly found that aqueous personal care rinse off compositions of the present invention including a

suds enhancing structurant; wherein the suds enhancing structurant comprises a crosslinked cellulose ether containing 0.1 to 1.0 wt%, based on weight of the crosslinked cellulose ether, of polyether groups; exhibits a luxuriant feel having high zero shear viscosity (preferably, ≥ 20 Pa·s; more preferably, ≥ 25 Pa·s; most preferably, ≥ 30 Pa·s measured at 25 °C according to ASTM Standard D4287-00); a low shear viscosity at 10 $s^{-}$ (preferably, ≤ 5 Pa·s; more preferably, ≤ 4 Pa·s; most preferably, ≤ 3.5 Pa·s measured at 25 °C according to ASTM Standard D4287-00) and a filament break-up time of ≤ 5 seconds (preferably, ≤ 4 seconds; more preferably, ≤ 3 seconds). We have also surprisingly found that the suds enhancing structurant also enhances and prolongs the suds life of the composition.

Unless otherwise indicated, ratios, percentages, parts, and the like are by weight.

As used herein, unless otherwise indicated, the phrase "molecular weight" or Mw refers to the weight average molecular weight as measured in a conventional manner with gel permeation chromatography (GPC) and poly(ethylene oxide) standards. GPC techniques are discussed in detail in Modern Size Exclusion Chromatography, W. W. Yau, J. J. Kirkland, D. D. Bly; Wiley-Interscience, 1979, and in A Guide to Materials Characterization and Chemical Analysis, J. P. Sibilia; VCH, 1988, p.81-84. Molecular weights are reported herein in units of Daltons, or equivalently, g/mol.

The term "dermatologically acceptable" as used herein and in the appended refers to ingredients that are typically used for topical application to the skin, and is intended to underscore that materials that are toxic when present in the amounts typically found in skin care compositions are not contemplated as part of the present invention.

The term "sulfate-free" as used herein and in the appended claims means compositions containing ≤ 1 wt% (preferably, ≤ 0.5 wt%; more preferably, ≤ 0.2 wt%; still more preferably, ≤ 0.01 wt%; yet still more preferably, ≤ 0.001 wt%; most preferably, less than the detectable limit) of sulfate.

The term "DS" as used herein and in the appended claims means the number of alkyl substituted OH groups per anhydroglucose unit in a cellulose ether, as determined by the Zeisel Method.

The term "DS (methyl)" or "DS (M)" as used herein and in the appended claims means the number of methyl substituted OH groups per anhydroglucose unit in a cellulose ether, as determined by the Zeisel Method.

The term "MS" as used herein and in the appended claims means the number of moles of etherification reagent which are bound as ether per mol of anhydroglucose unit as hydroxyalkyl substituents in a cellulose ether, as determined by the Zeisel Method.

The term "MS (hydroxyethyl)" or "MS (HE)" as used herein and in the appended claims means the number of moles of etherification reagent which are bound as ether per mol of anhydroglucose unit as hydroxyethyl substituents in a cellulose ether, as determined by the Zeisel Method.

The term "MS (hydroxypropyl)" or "MS (HP)" as used herein and in the appended claims means the number of moles of etherification reagent which are bound as ether per mol of anhydroglucose unit as hydroxypropyl substituents in a cellulose ether, as determined by the Zeisel Method.

The term "Zeisel Method" refers to the Zeisel cleavage procedure for determination of MS and DS. See G. Bartelmus and R. Ketterer, Zeitschriftfuer Analytische Chemie, Vol. 286 (1977, Springer, Berline, DE), pages 161-190.

The term "aesthetic characteristics" as used herein and in the appended claims in reference to an aqueous personal care rinse off composition refers to visual and tactile sensory properties (e.g., smoothness, tack, lubricity, texture, color, clarity, turbidity, uniformity).

Preferably, the aqueous personal care rinse off composition of the present invention is selected from the group consisting of a shampoo, a conditioning shampoo, a body wash, an exfoliating body wash, a facial wash, an exfoliating facial wash, a liquid hand soap, a sulfate-free cleansing and a mild cleansing. More preferably, the aqueous personal care rinse off composition of the present invention is selected from the group consisting of a shampoo, a conditioning shampoo; body wash, a facial wash and a liquid hand soap. Most preferably, the aqueous personal care rinse off composition of the present invention is selected from the group consisting of a shampoo and a conditioning shampoo.

Preferably, the aqueous personal care rinse off composition of the present invention, comprises: a dermatologically acceptable aqueous vehicle (preferably, wherein the aqueous personal care rinse off composition comprises 25 to 99 wt% (preferably, 30 to 95 wt%; more preferably, 40 to 92 wt%; most preferably, 50 to 90 wt%), based on weight of the aqueous personal care rinse off composition, of a dermatologically acceptable aqueous vehicle); a dermatologically acceptable cleaning surfactant (preferably, wherein the aqueous personal care rinse off composition comprises 0.5 to 60 wt% (preferably, 1 to 50 wt%; more preferably, 5 to 20 wt%; most preferably, 7 to 15 wt%), based on weight of the aqueous personal care rinse off composition, of a dermatologically acceptable cleaning surfactant); and a suds enhancing structurant (preferably, wherein the aqueous personal care rinse off composition comprises 0.01 to 5 wt%; more preferably, 0.05 to 3 wt%; still more preferably, 0.075 to 2 wt%; most preferably, 0.1 to 1.5 wt%), based on weight of the aqueous personal care rinse off composition, of the suds enhancing structurant); wherein the suds enhancing structurant comprises a crosslinked cellulose ether containing 0.1 to 1.0 wt%, based on weight of the crosslinked cellulose ether, of polyether groups.

Preferably, the aqueous personal care rinse off composition of the present invention, comprises a dermatologically acceptable aqueous vehicle. More preferably, the aqueous personal care rinse off composition of the present invention, comprises: 25 to 99 wt% (preferably, 30 to 95 wt%; more preferably, 40 to 90 wt%; most preferably, 50 to 90 wt%),

based on weight of the aqueous personal care rinse off composition, of a dermatologically acceptable aqueous vehicle. Still more preferably, the aqueous personal care rinse off composition of the present invention, comprises: 25 to 99 wt% (preferably, 30 to 95 wt%; more preferably, 40 to 90 wt%; most preferably, 50 to 90 wt%), based on weight of the aqueous personal care rinse off composition, of a dermatologically acceptable aqueous vehicle; wherein the dermatologically acceptable aqueous vehicle comprises water. Yet more preferably, the aqueous personal care rinse off composition of the present invention, comprises: 25 to 99 wt% (preferably, 30 to 95 wt%; more preferably, 40 to 90 wt%; most preferably, 50 to 90 wt%), based on weight of the aqueous personal care rinse off composition, of a dermatologically acceptable aqueous vehicle; wherein the dermatologically acceptable aqueous vehicle is selected from the group consisting of water and an aqueous $C_{1-4}$ alcohol mixture. Most preferably, the aqueous personal care rinse off composition of the present invention, comprises: 25 to 99 wt% (preferably, 30 to 95 wt%; more preferably, 40 to 90 wt%; most preferably, 50 to 90 wt%), based on weight of the aqueous personal care rinse off composition, of a dermatologically acceptable aqueous vehicle, wherein the dermatologically acceptable aqueous vehicle is water.

Preferably, the water used in the aqueous personal care rinse off composition of the present invention is at least one of distilled water and deionized water. More preferably, the water used in the aqueous personal care rinse off composition of the present invention is distilled and deionized.

Preferably, the aqueous personal care rinse off composition of the present invention comprises 0.5 to 60 wt% (preferably, 1 to 50 wt%; more preferably, 5 to 20 wt%; most preferably, 7 to 15 wt%), based on weight of the aqueous personal care rinse off composition, of a dermatologically acceptable cleaning surfactant. More preferably, the aqueous personal care rinse off composition of the present invention comprises 0.5 to 60 wt% (preferably, 1 to 50 wt%; more preferably, 5 to 20 wt%; most preferably, 7 to 15 wt%), based on weight of the aqueous personal care rinse off composition, of a dermatologically acceptable cleaning surfactant; wherein the dermatologically acceptable cleaning surfactant is selected from the group consisting of alkyl polyglucosides (e.g., lauryl glucoside, coco-glucoside, decyl glucoside), fatty alcohols (e.g., cetearyl alcohol, stearyl alcohol, cetyl alcohol, lauryl alcohol), glycinates (e.g., sodium cocoyl glycinate), betaines (e.g., alkyl betaines such as trimethylglycine and cetyl betaine; and amido betaines such as cocamidopropyl betaine), quaternary ammonia compounds (e.g., behentrimonium chloride, cetrimonium chloride), taurates (e.g., sodium methyl cocoyl taurate), glutamates (e.g., sodium cocoyl glutamate), sarcosinates (e.g., sodium lauroyl sarcosinate), isethionates (e.g., sodium cocoyl isethionate, sodium lauroyl methyl isethionate), sulfoacetates (e.g., sodium lauryl sulfoacetate), alaninates (e.g., sodium cocoyl alaninate), amphoacetates (e.g., sodium cocoamphoacetate), sulfates (e.g., sodium laureth sulfate (SLES)), sulfonates (e.g., sodium $C_{14-16}$ olefin sulfonate), succinates (e.g., disodium lauryl sulfosuccinate), fatty alkanolamides (e.g., cocamide monoethanolamine, cocamide, diethanolamine, soyamide diethanolamine, lauramide diethanolamine, oleamide monoisopropanolamine, stearamide monoethanolamine, myristamide monoethanolamine, lauramide monoethanolamine, capramide diethanolamine, ricinoleamide diethanolamine, myristamide diethanolamine, stearamide diethanolamine, oleylamide diethanolamine, tallowamide diethanolamine, lauramide monoisopropanolamine, tallowamide monoethanolamine, isostearamide diethanolamine, isostearamide diethanolamine, isostearamide monoethanolamine) and mixtures thereof. Yet more preferably, the aqueous personal care rinse off composition of the present invention comprises 0.5 to 60 wt% (preferably, 1 to 50 wt%; more preferably, 5 to 20 wt%; most preferably, 7 to 15 wt%), based on weight of the aqueous personal care rinse off composition, of a dermatologically acceptable cleaning surfactant; wherein the dermatologically acceptable cleaning surfactant is selected from the group consisting of alkyl polyglucosides (e.g., lauryl glucoside, coco-glucoside, decyl glucoside), glycinates (e.g., sodium cocoyl glycinate), betaines (e.g., alkyl betaines such as trimethylglycine and cetyl betaine; and amido betaines such as cocamidopropyl betaine), taurates (e.g., sodium methyl cocoyl taurate), glutamates (e.g., sodium cocoyl glutamate), sarcosinates (e.g., sodium lauroyl sarcosinate), isethionates (e.g., sodium cocoyl isethionate, sodium lauroyl methyl isethionate), sulfoacetates (e.g., sodium lauryl sulfoacetate), alaninates (e.g., sodium cocoyl alaninate), amphoacetates (e.g., sodium cocoamphoacetate), sulfates (e.g., sodium laureth sulfate (SLES)), sulfonates (e.g., sodium $C_{14-16}$ olefin sulfonate), succinates (e.g., disodium lauryl sulfosuccinate), fatty alkanolamides (e.g., cocamide monoethanolamine, cocamide, diethanolamine, soyamide diethanolamine, lauramide diethanolamine, oleamide monoisopropanolamine, stearamide monoethanolamine, myristamide monoethanolamine, lauramide monoethanolamine, capramide diethanolamine, ricinoleamide diethanolamine, myristamide diethanolamine, stearamide diethanolamine, oleylamide diethanolamine, tallowamide diethanolamine, lauramide monoisopropanolamine, tallowamide monoethanolamine, isostearamide diethanolamine, isostearamide diethanolamine, isostearamide monoethanolamine) and mixtures thereof. Still more preferably, the aqueous personal care rinse off composition of the present invention comprises 0.5 to 60 wt% (preferably, 1 to 50 wt%; more preferably, 5 to 20 wt%; most preferably, 7 to 15 wt%), based on weight of the aqueous personal care rinse off composition, of a dermatologically acceptable cleaning surfactant; wherein the dermatologically acceptable cleaning surfactant includes sodium laureth sulfate (SLES). Most preferably, the aqueous personal care rinse off composition of the present invention comprises 0.5 to 60 wt% (preferably, 1 to 50 wt%; more preferably, 5 to 20 wt%; most preferably, 7 to 15 wt%), based on weight of the aqueous personal care rinse off composition, of a dermatologically acceptable cleaning surfactant; wherein the dermatologically acceptable cleaning surfactant is a mixture of sodium laureth sulfate (SLES), cocamide monoethanolamine and cocamidopropyl betaine.

Preferably, the aqueous personal care rinse off composition of the present invention, comprises: 0.01 to 5 wt% (preferably, 0.05 to 3 wt%; more preferably, 0.075 to 2 wt%; most preferably, 0.1 to 1.5 wt%), based on weight of the aqueous personal care rinse off composition, of a suds enhancing structurant; wherein the suds enhancing structurant comprises a crosslinked cellulose ether containing 0.1 to 1.0 wt%, based on weight of the crosslinked cellulose ether, of polyether groups. More preferably, the aqueous personal care rinse off composition of the present invention, comprises: 0.01 to 5 wt% (preferably, 0.05 to 3 wt%; more preferably, 0.075 to 2 wt%; most preferably, 0.1 to 1.5 wt%), based on weight of the aqueous personal care rinse off composition, of a suds enhancing structurant; wherein the suds enhancing structurant comprises a crosslinked cellulose ether containing 0.1 to 1.0 wt%, based on weight of the crosslinked cellulose ether, of polyether groups; wherein the crosslinked cellulose ether comprises a base cellulose ether and crosslinks; wherein the crosslinks contain the polyether groups and wherein the base cellulose ether is a mixed cellulose ether containing hydroxyalkyl ether groups and alkyl ether groups. Still more preferably, the aqueous personal care rinse off composition of the present invention, comprises: 0.01 to 5 wt% (preferably, 0.05 to 3 wt%; more preferably, 0.075 to 2 wt%; most preferably, 0.1 to 1.5 wt%), based on weight of the aqueous personal care rinse off composition, of a suds enhancing structurant; wherein the suds enhancing structurant comprises a crosslinked cellulose ether containing 0.1 to 1.0 wt%, based on weight of the crosslinked cellulose ether, of polyether groups; wherein the crosslinked cellulose ether comprises a base cellulose ether and crosslinks; wherein the crosslinks contain the polyether groups and wherein the base cellulose ether is selected from the group consisting of hydroxyethyl methylcellulose, hydroxypropyl methyl cellulose, methyl hydroxyethyl hydroxypropylcellulose, ethyl hydroxyethyl cellulose and combinations thereof. Most preferably, the aqueous personal care rinse off composition of the present invention, comprises: 0.01 to 5 wt% (preferably, 0.05 to 3 wt%; more preferably, 0.075 to 2 wt%; most preferably, 0.1 to 1.5 wt%), based on weight of the aqueous personal care rinse off composition, of a suds enhancing structurant; wherein the suds enhancing structurant comprises a crosslinked cellulose ether containing 0.1 to 1.0 wt%, based on weight of the crosslinked cellulose ether, of polyether groups; wherein the crosslinked cellulose ether comprises a base cellulose ether and crosslinks; wherein the crosslinks contain the polyether groups and wherein the base cellulose ether is hydroxyethyl methylcellulose.

Preferably, the aqueous personal care rinse off composition of the present invention, comprises: 0.01 to 5 wt% (preferably, 0.05 to 3 wt%; more preferably, 0.075 to 2 wt%; most preferably, 0.1 to 1.5 wt%), based on weight of the aqueous personal care rinse off composition, of a suds enhancing structurant; wherein the suds enhancing structurant comprises a crosslinked cellulose ether containing 0.1 to 1.0 wt%, based on weight of the crosslinked cellulose ether, of polyether groups and wherein the crosslinked cellulose either is an irreversibly crosslinked cellulose ether. More preferably, the aqueous personal care rinse off composition, comprises: 0.01 to 5 wt% (preferably, 0.05 to 3 wt%; more preferably, 0.075 to 2 wt%; most preferably, 0.1 to 1.5 wt%), based on weight of the aqueous personal care rinse off composition, of a suds enhancing structurant; wherein the suds enhancing structurant comprises a crosslinked cellulose ether containing 0.1 to 1.0 wt%, based on weight of the crosslinked cellulose ether, of polyether groups; wherein the crosslinked cellulose ether comprises a base cellulose ether and crosslinks; wherein the crosslinks contain the polyether groups; wherein the base cellulose ether is a mixed cellulose ether containing hydroxyalkyl ether groups and alkyl ether groups and wherein the crosslinked cellulose either is an irreversibly crosslinked cellulose ether. Still more preferably, the aqueous personal care rinse off composition of the present invention, comprises: 0.01 to 5 wt% (preferably, 0.05 to 3 wt%; more preferably, 0.075 to 2 wt%; most preferably, 0.1 to 1.5 wt%), based on weight of the aqueous personal care rinse off composition, of a suds enhancing structurant; wherein the suds enhancing structurant comprises a crosslinked cellulose ether containing 0.1 to 1.0 wt%, based on weight of the crosslinked cellulose ether, of polyether groups; wherein the crosslinked cellulose ether comprises a base cellulose ether and crosslinks; wherein the crosslinks contain the polyether groups; wherein the base cellulose ether is selected from the group consisting of hydroxyethyl methylcellulose, hydroxypropyl methyl cellulose, methyl hydroxyethyl hydroxypropylcellulose, ethyl hydroxyethyl cellulose and combinations thereof and wherein the crosslinked cellulose either is an irreversibly crosslinked cellulose ether. Most preferably, the aqueous personal care rinse off composition of the present invention, comprises: 0.01 to 5 wt% (preferably, 0.05 to 3 wt%; more preferably, 0.075 to 2 wt%; most preferably, 0.1 to 1.5 wt%), based on weight of the aqueous personal care rinse off composition, of a suds enhancing structurant; wherein the suds enhancing structurant comprises a crosslinked cellulose ether containing 0.1 to 1.0 wt%, based on weight of the crosslinked cellulose ether, of polyether groups; wherein the crosslinked cellulose ether comprises a base cellulose ether and crosslinks; wherein the crosslinks contain the polyether groups; wherein the base cellulose ether is hydroxyethyl methylcellulose and wherein the crosslinked cellulose either is an irreversibly crosslinked cellulose ether.

Preferably, the crosslinked cellulose ether contains 0.1 to 1.0 wt% (preferably, 0.1 to 0.6 wt%; more preferably, 0.12 to 0.45 wt%; most preferably, 0.12 to 0.29 wt%), based on weight of the crosslinked cellulose ether, of polyether groups. More preferably, the crosslinked cellulose ether contains 0.1 to 1.0 wt% (preferably, 0.1 to 0.6 wt%; more preferably, 0.12 to 0.45 wt%; most preferably, 0.12 to 0.29 wt%), based on weight of the crosslinked cellulose ether, of polyether groups; wherein the polyether groups are polyoxyalkylene groups having 2 to 100 (preferably, 2 to 20; more preferably, 3 to 15) oxyalkylene groups per crosslink. Most preferably, the crosslinked cellulose ether contains 0.1 to 1.0 wt% (preferably, 0.1 to 0.6 wt%; more preferably, 0.12 to 0.45 wt%; most preferably, 0.12 to 0.29 wt%), based on weight of

the crosslinked cellulose ether, of polyether groups; wherein the polyether groups are polyoxypropylene groups having 2 to 100 (preferably, 2 to 20; more preferably, 3 to 15) oxypropylene groups per crosslink.

Preferably, crosslinked cellulose ether comprises a base cellulose ether having crosslinks containing 0.1 to 1.0 wt%, based on weight of the crosslinked cellulose ether, of polyether groups. Preferably, the base cellulose ether is selected from hydroxyalkyl cellulose ethers, alkyl cellulose ethers and combinations thereof. Examples of base cellulose ethers include, for example, methylcellulose, ethylcellulose, propylcellulose, butylcellulose, hydroxyethyl methylcellulose, hydroxypropyl methylcellulose, hydroxyethylcellulose, ethylhydroxyethylcellulose, methylethylhydroxyethylcellulose, hydrophobically modified ethylhydroxyethylcellulose, hydrophobically modified hydroxyethylcellulose, sulfoethyl methylhydroxyethylcellulose, sulfoethyl methylhydroxypropylcellulose and sulfoethyl hydroxyethylcellulose. Preferably, the base cellulose ethers are mixed cellulose ethers that contain both hydroxyalkyl ether groups and alkyl ether groups, such as, alkyl hydroxyethyl cellulose and hydroxyalkyl methylcellulose (e.g., hydroxyethyl methylcellulose, hydroxypropyl methylcellulose, methyl hydroxyethyl hydroxypropylcellulose and ethylhydroxyethyl cellulose).

Preferably, the base cellulose ether contains hydroxyalkyl ether substitutions. More preferably, the base cellulose ether has a degree of hydroxyethyl ether substitutions, MS (HE), or hydroxypropyl ether substitutions, MS (HP), of 1.5 to 4.5 (preferably, 2.0 to 3.0).

Preferably, the base cellulose ether contains methyl ether substitutions. More preferably, the base cellulose ether has a degree of methyl ether substitution, DS (M), of 1.2 to 2.1 (preferably, 1.3 to 1.7; more preferably, 1.35 to 1.60).

Preferably, the base cellulose ether is a mixed cellulose ether containing hydroxyalkyl ether substitutions and alkyl ether substitutions. More preferably, the base cellulose ether is a mixed cellulose ether having a degree of hydroxyethyl ether substitution, MS (HE), of 0.05 to 0.75 (preferably, 0.15 to 0.45; more preferably, 0.20 to 0.40) and a degree of methyl ether substitution, DS (M), of 1.2 to 2.1 (preferably, 1.3 to 1.7, more preferably, 1.35 to 1.60).

Preferably, the base cellulose ether is a mixed cellulose ether containing hydroxyalkyl ether substitutions and alkyl ether substitutions. More preferably, the base cellulose ether is a mixed cellulose ether having a degree of hydroxypropyl ether substitution, MS (HP), of 0.1 to 1.5 (preferably, 0.2 to 1.2) and a degree of methyl ether substitution, DS (M), of 1.2 to 2.1 (preferably, 1.3 to 2.0).

Preferably, the crosslinked cellulose ether comprises a base cellulose ether having crosslinks containing 0.1 to 1.0 wt%, based on weight of the crosslinked cellulose ether, of polyether groups; wherein the base cellulose ether is a hydroxyethyl methyl cellulose and wherein the crosslinks are polyoxypropylene dioxyethylene ether crosslinks, such as those produced as the reaction product of hydroxyethyl methyl cellulose with polypropylene glycol (PPG) glycidylether.

Crosslinking agents used to crosslink the base cellulose ether to form the crosslinked cellulose ether include compounds having a polyoxyalkylene or polyalkylene glycol group and two or more (preferably, two) crosslinking groups, such as, halogen groups, glycidyl or epoxy groups, and ethylenically unsaturated groups (e.g., vinyl groups) that form ether bonds with the base cellulose ether to form the crosslinked cellulose ether. Preferably, the crosslinking agent is selected from the group consisting of 1,2-dichloro(poly)alkoxy ethers, dichloropolyoxyethylene, diglycidyl polyalkoxy ethers, diglycidyl phosphonate, divinyl polyoxyalkylenes containing a sulphone group. Crosslinking agents having two different types of functional groups can be used. Examples include diglycidyl polyoxypropylenes and glycidyl(poly)oxyalkyl methacrylate. Preferably, the crosslinking agent contains 2 to 100 (preferably, 2 to 20; more preferably, 3 to 15) oxyalkylene groups per molecule.

Preferably, the amount of crosslinking agent included in the crosslinked cellulose ether ranges from 0.0001 to 0.05 eq (preferably, 0.0002 to 0.01 eq; more preferably, 0.0004 to 0.005 eq; most preferably, 0.0005 to 0.003 eq), wherein the unity "eq" represents the molar ratio of moles of the crosslinking agent relative to the number of moles of anhydroglucose units (AGU) in the base cellulose ether.

Preferably, the crosslinked cellulose ether is an irreversibly crosslinked cellulose ether. That is, the crosslinks in the crosslinked cellulose ether do not break down during the intended use of the crosslinked cellulose ether under normal conditions. In contrast, reversible crosslinks will break down during the intended use of the crosslinked cellulose ether under normal conditions. An example of reversible crosslinks in cellulose ethers intended for use in personal care rinse off compositions are those created using aldehyde based crosslinkers (e.g., glyoxal), which crosslinks break down upon dissolution of the crosslinked material in water.

Preferably, the aqueous personal care rinse off composition of the present invention, optionally, further comprises at least one additional ingredient selected from the group consisting of an absorbent; an aesthetic enhancer (e.g., starch); an alpha hydroxy acid; an antiaging agent; an antifungal; an antimicrobial agent; an antioxidant (e.g., butylated hydroxytoluene); an antiseptic; an antistatic agent; a bioactive agent; a bleaching agent; a chelating agent; a colorant; a conditioning agent (e.g., silicone, polyquaternium); a dye; an emollient; an emulsifying agent; a film former (e.g., water proofing agent); a fixative polymer; a foaming agent; a fragrance; a hard particle; a humectant (e.g., glycerin, sorbitol, monoglycerides, lecithins, glycolipids, fatty alcohols, fatty acids, polysaccharides, sorbitan esters, polysorbates (e.g., Polysorbate 20, Polysorbate 40, Polysorbate 60, and Polysorbate 80), diols (e.g., propylene glycol), diol analogs, triols, triol analogs, cationic polymeric polyols); a lubricating agent; an opacifier; a pearlizing agent; a penetrant; a pH adjusting agent; a pigment; a plant extract; a preservative (e.g., benzoic acid, sorbic acid, phenoxyethanol); a protein/amino acid; a

rheology modifier; a salt; a sensory modifier; a slip agent; a soft particle; a sunscreen additive; a UV light inhibitor; a vitamin and mixtures thereof. More preferably, the skin cleansing formulation of the present invention, optionally, further comprises at least one additional ingredient selected from the group consisting of at least one of a preservative, a rheology modifier and a pH adjusting agent.

Preferably, the aqueous personal care rinse off composition of the present invention, further comprises a rheology modifier. More preferably, the aqueous personal care rinse off formulation of the present invention, further comprises a rheology modifier; wherein the rheology modifier is selected to increase the viscosity of the aqueous personal care rinse off composition (preferably without substantially modifying the other properties of the aqueous personal care rinse off composition). Still more preferably, the aqueous personal care rinse off composition of the present invention, further comprises 0 to 10 wt% (preferably, 0.05 to 5 wt%; more preferably, 0.1 to 2.5 wt%; most preferably, 0.15 to 2.0 wt%), based on weight of the aqueous personal care rinse off composition, of a rheology modifier. Most preferably, the aqueous personal care rinse off composition of the present invention, further comprises 0 to 10 wt% (preferably, 0.05 to 5 wt%; more preferably, 0.1 to 2.5 wt%; most preferably, 0.15 to 2.0 wt%), based on weight of the aqueous personal care rinse off composition, of a rheology modifier; wherein the rheology modifier is selected from the group consisting of sodium chloride, cellulose, a modified cellulose, xanthan gum, an acrylates copolymer and mixtures thereof.

Preferably, the aqueous personal care rinse off composition of the present invention, further comprises an antimicrobial agent. More preferably, the aqueous personal care rinse off composition of the present invention, further comprises an antimicrobial agent; wherein the antimicrobial agent is selected from the group consisting of phenoxyethanol, benzoic acid, benzyl alcohol, sodium benzoate, DMDM hydantoin, 2-ethylhexyl glyceryl ether and isothiazolinone (e.g., methylchloroisothiazolinone, methylisothiazolinone). Still more preferably, the aqueous personal care rinse off composition of the present invention, further comprises an antimicrobial agent; wherein the antimicrobial agent is an isothiazolinone (more preferably, wherein the antimicrobial is selected from the group consisting of methylisothiazolinone, methylchloroisothiazolinone and mixtures thereof; most preferably, wherein the biocide is methylisothiazolinone). Most preferably, the aqueous personal care rinse off composition of the present invention, further comprises an antimicrobial agent; wherein the antimicrobial agent is an isothiazolinone (more preferably, wherein the antimicrobial agent is selected from the group consisting of methylisothiazolinone, methylchloroisothiazolinone and mixtures thereof; most preferably, wherein the antimicrobial agent is methylisothiazolinone); and wherein the aqueous personal care rinse off composition is a body wash formulation.

Preferably, the aqueous personal care rinse off composition of the present invention, further comprises a pH adjusting agent. More preferably, the aqueous personal care rinse off composition of the present invention, further comprises a pH adjusting agent; wherein the aqueous personal care rinse off composition is a body wash formulation. Most preferably, the aqueous personal care rinse off composition of the present invention, further comprises a pH adjusting agent; wherein the aqueous personal care rinse off composition is a body wash formulation and wherein the body wash formulation has a pH of 4.5 to 9 (preferably, 5 to 8; most preferably, 6 to 7).

Preferably, the pH adjusting agent is selected from the group consisting of at least one of citric acid, lactic acid, hydrochloric acid, aminoethyl propanediol, triethanolamine, monoethanolamine, sodium hydroxide, potassium hydroxide, amino-2-methyl-1-propanol. More preferably, the pH adjusting agent is selected from the group consisting of at least one of citric acid, lactic acid, sodium hydroxide, potassium hydroxide, triethanolamine, amino-2-methyl-1-propanol. Still more preferably, the pH adjusting agent includes is triethanolamine. Most preferably, the pH adjusting agent is triethanolamine.

Preferably, the aqueous personal care rinse off composition of the present invention, further comprises a colorant. More preferably, the aqueous personal care rinse off composition of the present invention, further comprises a colorant; wherein the aqueous personal care rinse off composition is selected from the group consisting of a shampoo, a conditioning shampoo; body wash, a facial wash and a liquid hand soap.

Preferably, the method of cleaning at least one of mammalian skin and hair of the present invention, comprises: applying an aqueous personal care rinse off composition of the present invention to the skin or hair of a mammal; and rinsing the aqueous personal care rinse off composition from the skin or hair with a rinse water.

Some embodiments of the present invention will now be described in detail in the following Examples.

## Synthesis 1: Crosslinked Cellulose Ether

The crosslinking agent used in Synthesis 1 was a linear poly(propyleneglycol) diglycidyl ether made from polypropylene glycol (PPG) having a molecular weight of ~400 Daltons and having the formula

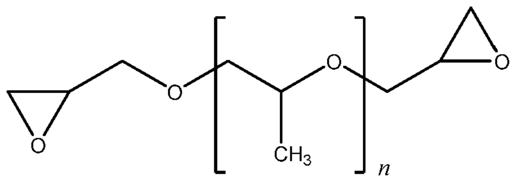

wherein n is 5.7 to 6.7 (available from Leuna-Harze GmbH, Leuna, DE as EPILOX™ M985 poly(propyleneglycol) diglycidylether crosslinker).

Ground cellulose flock (1.5 mol) was added to a 5 L autoclave. After purging the autoclave trice with nitrogen gas, the contents of the autoclave were heated to 40 °C. Then dimethylether (DME, 4.7 mol/mol of anhydroglucose units (AGU)) and methyl chloride (MCl; 3.2 mol/mol AGU) were injected into the autoclave. Caustic soda (NaOH, strength 50 wt% aqueous, 1.9 mol NaOH/mol AGU) was added to the autoclave in 3 portions during 2 minutes at a temperature of 40 °C. The reaction mixture was held at 40 °C for 30 minutes. Ethylene oxide (0.45 mol/mol AGU) was then added and the reaction mixture was held for 10 minutes at 40 °C. The crosslinker (EPILOX™ M985 crosslinker; 0.0012 mol/mol AGU) was dissolved in 20 mL of isopropanol and added to the contents of the autoclave in six increments in 30 second intervals. The contents of the autoclave were then heated to 80 °C in 45 minutes. At 80 °C a water soluble monovalent copper ligand (MCL 2; 1.3 mol/mol AGU) was injected into the autoclave quickly. Afterwards, NaOH (0.67 mol/mol AGU) was added in 7 portions over 30 minutes, followed by a 70 minute cook-off time at 80 °C. Following this, the product crosslinked cellulose ether was washed in hot (> 95 °C) water, neutralized with formic acid, granulated, dried and milled.

__Synthesis 2: Crosslinked Cellulose Ether__

The process of **Synthesis 1** was repeated in Synthesis 2, with the exception of the crosslinker loading was 0.0013 mol/mol AGU).

__Synthesis 3: Hydrophobically Modified Hydroxyethyl Cellulose Ether__

A 500 ml resin kettle was fitted with a mechanical stirring paddle, a nitrogen inlet, a rubber serum cap, and a reflux condenser connected to a mineral oil bubbler. The resin kettle was charged with 33.28 g (30.00 g contained) Biofloc XV wood pulp (available from Tembec), 173 g of isopropyl alcohol, and 27 g of distilled water. While stirring the mixture, the resin kettle was purged with nitrogen for one hour to remove any entrained oxygen in the system. While stirring under nitrogen, 4.00 g of 50% aqueous sodium hydroxide solution were added dropwise over five minutes using a syringe. The mixture was then allowed to stir for 30 minutes under nitrogen.

A solution of 4.00 g of 1-bromohexadecane in 10 mL of isopropyl alcohol was added by Syringe to the mixture under nitrogen (0.19 moles of 1-bromohexadecane per mole of HEC). Heat was then applied using a heating mantle, and the stirred mixture was heated at reflux for 4.5 hours under nitrogen. The mixture was then cooled to room temperature and neutralized by adding 5.00 g of glacial acetic acid and stirring for 10 minutes. The polymer was recovered by vacuum filtration and washed in a Waring blender: five times with 250 mL of 4:1 (by volume) of acetone/water and twice with 250 mL of pure acetone. The polymer was glyoxal-treated by adding 0.40 g of 40% aqueous glyoxal and 0.25 g of glacial acetic acid to the last acetone desiccation. The polymer was dried in vacuo at 50° C. overnight. The 1% aqueous viscosity of HmHEC polymer (corrected for ash and volatiles) was measured at 25.0° C. and found to be 16,000 mPa-sec (Brookfield LVT, 30 rpm, spindle #4). The ethylene oxide MS (EOMS) was found to be 1.88 and the hexadecyl DS was found to be 0.0059 by Zeisel analysis.

__Comparative Examples CF1-CF4 and Examples F1-F2: Shampoo Formulations__

Shampoo formulations were prepared having formulations according to **Comparative Examples CF1-CF4** and **Examples F1-F2** as noted in **TABLE 1.** The final pH of the product formulations were adjusted to a pH of 6 using citric acid or 2-amino-2-methylpropanol as necessary and sufficient water was added to adjust the total formulation weight to 100 g.

__TABLE 1__

| | CF1 | CF2 | CF3 | CF4 | F1 | F2 |
|---|---|---|---|---|---|---|
| __Ingredient INCI name__ | __wt%__ | __wt%__ | __wt%__ | __wt%__ | __wt%__ | __wt%__ |
| Deionized water | q.s. 100 | | | | | |
| 30% aq soln Sodium Lauryl Ether Sulfate[1] | 30 | 30 | 30 | 30 | 30 | 30 |

(continued)

| Ingredient INCI name | CF1 wt% | CF2 wt% | CF3 wt% | CF4 wt% | F1 wt% | F2 wt% |
|---|---|---|---|---|---|---|
| Cocamide MEA[2] | 1 | 1 | 1 | 1 | 1 | 1 |
| 30% aq soln Cocamidopropyl Betaine[3] | 6 | 6 | 6 | 6 | 6 | 6 |
| Hydroxyethyl cellulose[4] | 0.9 | -- | -- | -- | -- | -- |
| **Product Synthesis S3** | -- | 0.9 | -- | -- | -- | -- |
| Hydroxyethyl methyl celluloses | -- | -- | 0.9 | -- | -- | -- |
| Hydroxyethyl methyl cellulose[6] | -- | -- | -- | 0.9 | -- | -- |
| **Product Synthesis S1** | -- | -- | -- | -- | 0.9 | -- |
| **Product Synthesis S2** | -- | -- | -- | -- | -- | 0.9 |
| Tetrasodium EDTA[7] | 0.2 | 0.2 | 0.2 | 0.2 | 0.2 | 0.2 |
| Phenoxyethanol[8] | 0.5 | 0.5 | 0.5 | 0.5 | 0.5 | 0.5 |

[1] available from Pilot Chemical
[2] available from Croda Inc.
[3] available from Solvay
[4] available from The Dow Chemical Company under tradename CELLOSIZE™ QP-100MH
[5] available from The Dow Chemical Company under tradename WALOCEL™ MKX 70,000 PP 01
[6] available from The Dow Chemical Company under tradename WALOCEL™ VP-M 27149
[7] available from The Dow Chemical Company under tradename VERSENE™ 220
[8] available from DuPont Chemical Company under tradename NEOLONE PH-100

**Examples F3-F6: Conditioning Shampoo Formulations**

Conditioning shampoo formulations were prepared having formulations according to **Examples F3-F6** as noted in **TABLE 2.** The final pH of the product formulations were adjusted to a pH of 6 using citric acid or 2-amino-2-methylpropanol as necessary and sufficient water was added to adjust the total formulation weight to 100 g.

**TABLE 2**

| Ingredient INCI name | F3 wt% | F4 wt% | F5 wt% | F6 wt% |
|---|---|---|---|---|
| Deionized water | q.s. 100 | | | |
| 30% aq soln Sodium Lauryl Ether Sulfate[l] | 30 | 30 | 30 | 30 |
| Cocamide MEA[2] | 1 | 1 | 1 | 1 |
| 30% aq soln Cocamidopropyl Betaine[3] | 6 | 6 | 6 | 6 |
| Polyquaternium-10[4] | 0.1 | 0.1 | 0.1 | 0.1 |
| Cationic silicon microemulsions | 0.5* | -- | -- | -- |
| Silicone emulsion[6] | -- | 0.5* | -- | -- |
| Silicone polyether emulsion[7] | -- | -- | 0.5* | -- |
| Cationic silicon emulsion[8] | -- | -- | -- | 0.5* |
| **Product Synthesis S1** | 0.9 | 0.9 | 0.9 | 0.9 |
| Tetrasodium EDTA[9] | 0.2 | 0.2 | 0.2 | 0.2 |
| Phenoxyethanol | 0.5 | 0.5 | 0.5 | 0.5 |
| 1:1 wt ratio of Fragrance oil in PEG 3000[11] | 0.5 | 0.5 | 0.5 | 0.5 |

[1] available from Pilot Chemical

(continued)

| Ingredient INCI name | F3 wt% | F4 wt% | F5 wt% | F6 wt% |
|---|---|---|---|---|
| 2 available from Croda Inc.<br>3 available from Solvay<br>4 available from The Dow Chemical Company under tradename UCARE™ LR-400<br>5 Amodimethicone (and) $C_{11-15}$ pareth-7 (and) laureth-9 (and) glycerin (and) trideceth-12 available from The Dow Chemical Company under the tradename DOWSIL™ CE-8170 AF Microemulsion<br>6 Dimethiconol (and) TEA-dodecylbenzenesulfonate available from The Dow Chemical Company under the tradename XIAMETER™ MEM-1785 emulsion<br>7 Bis-diisopropanolamino-PG-propyl dimethicone/bis-isobutyl PEG-14 copolymer (and) polysorbate 20 (and) butyloctanol available from The Dow Chemical Company under the tradename DOWSIL™ CE-8411 smooth plus emulsion<br>8 Amodimethicone (and) cetrimonium chloride (and) trideceth-3 (and) trideceth-15 available from The Dow Chemical Company under the tradename DOWSIL™ 969 emulsion<br>9 available from The Dow Chemical Company under tradename VERSENE™ 220<br>10 available from DuPont Chemical Company under tradename NEOLONE PH-100<br>11 available from BrambleBerry<br>* silicone solids | | | | |

**Comparative Examples CF5-CF8 and Examples F7-F9: Silicone Free Conditioners**

Silicone free conditioner formulations were prepared having formulations according to **Comparative Examples CF5-CF8** and **Examples F7-F9** as noted in **TABLE 3.** The final pH of the product formulations were adjusted to a pH of 6 using citric acid or 2-amino-2-methylpropanol as necessary and sufficient water was added to adjust the total formulation weight to 100 g.

**TABLE 3**

| Ingredient INCI name | CF5 wt% | CF6 wt% | CF7 wt% | CF8 wt% | F7 wt% | F8 wt% | F9 wt% |
|---|---|---|---|---|---|---|---|
| Deionized water | q.s. 100 | | | | | | |
| Cetearyl alcohol[1] | 1.5 | 1.5 | 1.5 | 1.5 | 1.5 | 1.5 | 1.5 |
| Behentrimonium chloride[2] | -- | -- | -- | -- | -- | -- | 0.75 |
| PEG-100 stearate & glyceryl stearate[3] | 1 | 1 | 1 | 1 | 1 | 1 | 1 |
| Hydroxyethyl cellulose[4] | 0.9 | -- | -- | -- | -- | -- | -- |
| **Product Synthesis S3** | -- | 0.9 | -- | -- | -- | -- | -- |
| Hydroxyethyl methyl celluloses | -- | -- | 0.9 | -- | -- | -- | -- |
| Hydroxyethyl methyl cellulose[6] | -- | -- | -- | 0.9 | -- | -- | -- |
| **Product Synthesis S1** | -- | -- | -- | -- | 0.9 | -- | 0.9 |
| **Product Synthesis S2** | -- | -- | -- | -- | --- | 0.9 | -- |
| Tetrasodium EDTA[7] | 0.2 | 0.2 | 0.2 | 0.2 | 0.2 | 0.2 | 0.2 |
| Phenoxyethanol[8] | 0.5 | 0.5 | 0.5 | 0.5 | 0.5 | 0.5 | 0.5 |
| 1 available from Croda Inc.<br>2 available from Croda Inc.<br>3 available from Croda Inc.<br>4 available from The Dow Chemical Company under tradename CELLOSIZE™ QP-100MH<br>5 available from The Dow Chemical Company under tradename WALOCEL™ MKX 70,000 PP 01<br>6 available from The Dow Chemical Company under tradename WALOCEL™ VP-M 27149 | | | | | | | |

(continued)

| Ingredient INCI name | CF5 wt% | CF6 wt% | CF7 wt% | CF8 wt% | F7 wt% | F8 wt% | F9 wt% |
|---|---|---|---|---|---|---|---|
| [7] available from The Dow Chemical Company under tradename VERSENE™ 220 | | | | | | | |
| [8] available from DuPont Chemical Company under tradename NEOLONE PH-100 | | | | | | | |

**Comparative Examples CF9-CF12 and Examples F10-F11: Sulfate Free Shampoo**

Sulfate free shampoo formulations were prepared having formulations according to **Comparative Examples CF9-CF12** and **Examples F10-F11** as noted in **TABLE 4.** The final pH of the product formulations were adjusted to a pH of 6 using citric acid or 2-amino-2-methylpropanol as necessary and sufficient water was added to adjust the total formulation weight to 100 g.

**TABLE 4**

| Ingredient INCI name | CF9 wt% | CF10 wt% | CF11 wt% | CF12 wt% | F10 wt% | F11 wt% |
|---|---|---|---|---|---|---|
| Deionized water | q.s. 100 | | | | | |
| 50% aq soln Decyl glucoside[1] | 8 | 8 | 8 | 6 | 6 | 6 |
| 40% aq soln Disodium laureth sulfosuccinate[2] | 10 | 10 | 10 | -- | -- | -- |
| 30% aq soln Cocamidopropyl betaine[3] | 16 | 16 | 16 | 10 | 10 | 10 |
| 30% aq soln Sodium lauroyl sarcosinate[4] | 5 | 5 | 5 | -- | -- | -- |
| Potassium cocoyl glycinate[5] | -- | -- | -- | 9.5 | 9.5 | 9.5 |
| Hydroxyethyl cellulose[6] | 1 | -- | -- | 1 | -- | -- |
| **Product Synthesis S3** | -- | 1 | -- | -- | 1 | -- |
| **Product Synthesis S1** | -- | -- | 1 | -- | -- | 1 |
| Polyquaternium-10[7] | 0.1 | 0.1 | 0.1 | 0.1 | 0.1 | 0.1 |
| Phenoxyethanol[s] | 0.5 | 0.5 | 0.5 | 0.5 | 0.5 | 0.5 |

[1] available from The Dow Chemical Company under tradename ECOSENSE™ 3000
[2] available from Solvay under tradename MACKANATE EL P
[3] available from Solvay under tradename MIRATAINE CAB AS
[4] available from Croda under tradename CRODASINIC LS30
[5] available from Shanghai Puji Biotechnology Co., Ltd.
[6] available from The Dow Chemical Company under tradename CELLOSIZE™ QP-100MH
[7] available from The Dow Chemical Company under tradename UCARE™ LR-400
[8] available from DuPont Chemical Company

**Performance Testing**

Several formulation rheology parameters were tested using a DHR-3 rheometer (TA Instruments, New Castle, DE) with a 40 mm parallel plate geometry according to ASTM Standard D4440-15 and ASTM Standard D4287-00). All tests were carried out at 25 °C using a fixed gap of 1 mm and a solvent trap filled with deionized water. Each sample was preconditioned for 30 s at 0.5 $s^{-1}$ steady shear and allowed to equilibrate for 3 min. An oscillatory amplitude sweep was done from 0.1 to 350% at constant frequency of 1 rad/s; an oscillatory frequency sweep from 0.05 to 100 rad/s was done at constant strain of 2.5%; and a shear rate sweep rate was done at 0.05 to 750 $s^{-1}$. All analysis was performed using TA Instruments TRIOS software v5.1.1. The G"/G' value was obtained from the amplitude in the linear region (< 5% strain). The crossover frequency was determined from the frequency sweep. The viscosity values were obtained from the shear rate sweep. The shear rate index and zero-shear viscosity values were determined by fitting a Cross-Williamson model to the shear rate data

$$n = \frac{n_0}{1 + (K * \dot{\gamma})^x}$$

wherein $n_0$ is the zero shear viscosity and x is the shear rate index. The results are provided in **TABLE 5.**

Filament break-up time testing was performed on **Comparative Examples CF1-CF2** and **Exampes F1** and **F5.** Samples were tested on a Thermo-Haake Capillary Breakup Extensional Rheometer (CaBER) using 6 mm diameter parallel plates with an initial separation of 3 mm. All tests were conducted at 22 °C. A 50 ms linear strike profile was chosen, and the thickness of the filament midpoint was monitored as a function of time. Samples were tested multiple times using fresh loadings of sample to ensure reproducible results. The average filament break-up times are reported in **TABLE 6.**

**TABLE 5**

| Formulation | G"/G' | Crossover frequency | Viscosity (Pa·s) | | Shear rate index | Zero Shear (Pa·s) |
|---|---|---|---|---|---|---|
| | | | $1\ s^{-1}$ | $10\ s^{-1}$ | | |
| **Comp. Ex. CF1** | -- | 15.1 | 2.1 | 1.0 | 0.69 | 3.6 |
| **Comp. Ex. CF2** | -- | 4.7 | 8.5 | 2.2 | 0.67 | 24.1 |
| **Comp. Ex. CF3** | 1.9 | 14.6 | 4.2 | 1.7 | 0.65 | 8.0 |
| **Comp. Ex. CF4** | 1.6 | 6.1 | 8.0 | 2.6 | 0.72 | 16.3 |
| **Comp. Ex. CF5** | -- | 9.5 | 12.4 | 3.7 | 0.70 | 32.4 |
| **Comp. Ex. CF6** | -- | none | 28.5 | 5.7 | 0.95 | 2010 |
| **Comp. Ex. CF7** | 1.9 | 15.0 | 9.1 | 3.3 | 0.70 | 18.3 |
| **Comp. Ex. CF8** | 1.8 | 8.8 | 12.7 | 4.3 | 0.72 | 26.7 |
| **Comp. Ex. CF10** | 1.2 | 28.0 | 0.8 | 0.3 | -- | -- |
| **Comp. Ex. CF12** | 1.2 | 3.9 | 4.2 | 1.6 | -- | -- |
| **Example F1** | 1.3 | 3.5 | 9.7 | 2.6 | 0.68 | 32.3 |
| **Example F2** | 1.1 | 2.0 | 12.7 | 3.2 | 0.71 | 49.4 |
| **Example F4** | -- | -- | 4.23 | 1.39 | -- | -- |
| **Example F5** | -- | -- | 2.94 | 1.04 | -- | -- |
| **Example F7** | 1.0 | 1.2 | 23.3 | 5.3 | 0.74 | 129.3 |
| **Example F8** | 1.0 | 0.9 | 24.8 | 5.4 | 0.74 | 135.5 |
| **Example F9** | -- | -- | 27.92 | 6.03 | -- | -- |
| **Example F11** | 1.4 | 3.8 | 16.4 | 4.8 | 0.7 | 44.89 |

**TABLE 6**

| Formulation | Filament Break-up time (s) |
|---|---|
| **Comparative Example CF1** | 2.5 |
| **Comparative Example CF2** | >6.0 |
| **Example F1** | 2.6 |
| **Example F5** | 2.4 |

## Claims

**1.** An aqueous personal care rinse off composition, comprising:

a dermatologically acceptable aqueous vehicle;
a dermatologically acceptable cleaning surfactant; and

a suds enhancing structurant; wherein the suds enhancing structurant comprises a crosslinked cellulose ether containing 0.1 to 1.0 wt%, based on weight of the crosslinked cellulose ether, of polyether groups.

**2.** The aqueous personal care rinse off composition of claim 1, wherein the aqueous personal care rinse off composition is selected from the group consisting of a shampoo, a conditioning shampoo, a body wash formulation, an exfoliating body wash formulation, a facial wash formulation, an exfoliating facial wash formulation, a liquid hand soap formulation, a sulfate-free cleansing formulation and a mild cleansing formulation.

**3.** The aqueous personal care rinse off composition of claim 2, wherein the crosslinked cellulose ether is an irreversibly crosslinked cellulose ether.

**4.** The aqueous personal care rinse off composition of claim 3, wherein the polyether groups in the irreversibly crosslinked cellulose ether are polyoxyalkylene groups having 2 to 100 oxyalkylene groups.

**5.** The aqueous personal care rinse off composition of claim 4, wherein the polyoxyalkylene groups are selected from the group consisting of a polyoxyethylene, a polyoxypropylene and combinations thereof.

**6.** The aqueous personal care rinse off composition of claim 5, wherein the irreversibly crosslinked cellulose ether comprises a base cellulose ether and crosslinks; wherein the crosslinks contain the polyether groups; and wherein the base cellulose ether is selected from the group consisting of hydroxyethyl methylcellulose, hydroxypropyl methyl cellulose, methyl hydroxyethyl hydroxypropylcellulose, ethyl hydroxyethyl cellulose and combinations thereof.

**7.** The aqueous personal care rinse off composition of claim 6, wherein the base cellulose ether is hydroxyethyl methylcellulose.

**8.** The aqueous personal care rinse off composition of claim 7, wherein the dermatologically acceptable cleaning surfactant is selected from the group consisting of alkyl polyglucosides, glycinates, betaines, taurates, glutamates, sarcosinates, isethionates, sulfoacetates, alaninates, amphoacetates, sulfates, sulfonates, succinates, fatty alkanolamides and mixtures thereof.

**9.** The aqueous personal care rinse off composition of claim 8, further comprising an optional ingredient selected from the group consisting of an absorbent, an aesthetic enhancer, an alpha hydroxy acid, an antiaging agent, an antifungal, an antimicrobial agent, an antioxidant, an antiseptic, an antistatic agent, a bioactive agent, a bleaching agent, a chelating agent, a colorant, a dye, an emollient, an emulsifying agent, a film former, a fixative polymer, a foaming agent, a fragrance, a hard particle, a humectant, a lubricating agent, an opacifier, a pearlizing agent, a penetrant, a pH adjusting agent, a pigment, a plant extract, a preservative, a protein/amino acid, a rheology modifier, a salt, a sensory modifier, a slip agent, a soap, a soft particle, a sunscreen additive, a UV light inhibitor, a vitamin and mixtures thereof.

**10.** A method of cleaning at least one of mammalian skin and hair, comprising:

(a) applying an aqueous personal care rinse off composition according to claim 1 to the skin or hair of a mammal; and
(b) rinsing the aqueous personal care rinse off composition from the skin or hair with a rinse water.

## Patentansprüche

**1.** Wässrige Körperpflegeabspülzusammensetzung, umfassend:

ein dermatologisch verträgliches wässriges Vehikel;
ein dermatologisch verträgliches Reinigungstensid; und
ein schaumverstärkendes Strukturierungsmittel; wobei das schaumverstärkende Strukturierungsmittel einen vernetzten Celluloseether umfasst, der zu 0,1 bis 1,0 Gew.-%, basierend auf dem Gewicht des vernetzten Celluloseethers, Polyethergruppen enthält.

**2.** Wässrige Körperpflegeabspülzusammensetzung nach Anspruch 1, wobei die wässrige Körperpflegeabspülzusammensetzung aus der Gruppe ausgewählt ist, bestehend aus einem Shampoo, einem Pflegeshampoo, einer Körperwaschformulierung, einer Peeling-Körperwaschformulierung, einer Gesichtswaschformulierung, einer Peeling-Gesichtswaschformulierung, einer flüssigen Handseifenformulierung, einer sulfatfreien Reinigungsformulierung und einer milden Reinigungsformulierung.

**3.** Wässrige Körperpflegeabspülzusammensetzung nach Anspruch 2, wobei der vernetzte Celluloseether ein irreversibel vernetzter Celluloseether ist.

**4.** Wässrige Körperpflegeabspülzusammensetzung nach Anspruch 3, wobei die Polyethergruppen in dem irreversibel vernetzten Celluloseether Polyoxyalkylengruppen sind, die 2 bis 100 Oxyalkylengruppen aufweisen.

**5.** Wässrige Körperpflegeabspülzusammensetzung nach Anspruch 4, wobei die Polyoxyalkylengruppen aus der Gruppe ausgewählt sind, bestehend aus einem Polyoxyethylen, einem Polyoxypropylen und Kombinationen davon.

**6.** Wässrige Körperpflegeabspülzusammensetzung nach Anspruch 5, wobei der irreversibel vernetzte Celluloseether einen Basiscelluloseether und Vernetzungen umfasst; wobei die Vernetzungen die Polyethergruppen enthalten; und wobei der Basiscelluloseether aus der Gruppe ausgewählt ist, bestehend aus Hydroxyethylmethylcellulose, Hydroxypropylmethylcellulose, Methylhydroxyethylhydroxypropylcellulose, Ethylhydroxyethylcellulose und Kombinationen davon.

**7.** Wässrige Körperpflegeabspülzusammensetzung nach Anspruch 6, wobei der Basiscelluloseether Hydroxyethylmethylcellulose ist.

**8.** Wässrige Körperpflegeabspülzusammensetzung nach Anspruch 7, wobei das dermatologisch verträgliche Reinigungstensid aus der Gruppe ausgewählt ist, bestehend aus Alkylpolyglucosiden, Glycinaten, Betainen, Tauraten, Glutamaten, Sarcosinaten, Isethionaten, Sulfoacetaten, Alaninaten, Amphoacetaten, Sulfaten, Sulfonaten, Succinaten, Fettalkanolamiden und Mischungen davon.

**9.** Wässrige Körperpflegeabspülzusammensetzung nach Anspruch 8, ferner umfassend einen optionalen Bestandteil, der aus der Gruppe ausgewählt ist, bestehend aus einem Absorptionsmittel, einem ästhetischen Verstärker, einer Alpha-Hydroxysäure, einem Anti-Aging-Mittel, einem Antimykotikum, einem antimikrobiellen Mittel, einem Antioxidans, einem Antiseptikum, einem Antistatikum, einem bioaktiven Mittel, einem Bleichmittel, einem Chelatbildner, einem Färbemittel, einem Farbstoff, einem Emolliens, einem Emulgierungsmittel, einem Filmbildner, einem Fixierpolymer, einem Schaumbildner, einem Duftstoff, einem harten Partikel, einem Feuchthaltemittel, einem Schmiermittel, einem Trübungsmittel, einem Perlglanzmittel, einem Eindringmittel, einem pH-Wert-Einstellmittel, einem Pigment, einem Pflanzenextrakt, einem Konservierungsmittel, einem Protein/einer Aminosäure, einem Rheologiemodifikator, einem Salz, einem sensorischen Modifikator, einem Gleitmittel, einer Seife, einem weichen Partikel, einem Sonnenschutzzusatz, einem UV-Licht-Inhibitor, einem Vitamin und Mischungen davon.

**10.** Verfahren zum Reinigen mindestens einem von Haut und Haar von Säugetieren, umfassend:

(a) Auftragen einer wässrigen Körperpflegeabspülzusammensetzung nach Anspruch 1 auf die Haut oder das Haar eines Säugetiers; und
(b) Abspülen der wässrigen Körperpflegeabspülzusammensetzung von der Haut oder dem Haar mit einem Spülwasser.

## Revendications

**1.** Composition aqueuse pour soins personnels à rincer, comprenant :

un véhicule aqueux dermatologiquement acceptable ;
un agent tensioactif de nettoyage dermatologiquement acceptable ; et
un structurant améliorant la mousse ; dans laquelle le structurant améliorant la mousse comprend un éther de cellulose réticulé contenant de 0,1 à 1,0 % en poids, sur la base du poids de l'éther de cellulose réticulé, de groupes polyéther.

**2.** Composition aqueuse de soins personnels à rincer selon la revendication 1, dans laquelle la composition aqueuse de soins personnels à rincer est choisie dans le groupe constitué d'un shampooing, d'un shampooing revitalisant, d'une formulation de lavage du corps, d'une formulation exfoliante de lavage du corps, d'une formulation de lavage du

visage, d'une formulation exfoliante de lavage du visage, d'une formulation de savon liquide pour les mains, d'une formulation de nettoyage sans sulfate et d'une formulation de nettoyage doux.

**3.** Composition aqueuse de soins personnels à rincer selon la revendication 2, dans laquelle l'éther de cellulose réticulé est un éther de cellulose irréversiblement réticulé.

**4.** Composition aqueuse de soins personnels à rincer selon la revendication 3, dans laquelle les groupes polyéther de l'éther de cellulose irréversiblement réticulé sont des groupes polyoxyalkylène ayant de 2 à 100 groupes oxyalkylène.

**5.** Composition aqueuse de soins personnels à rincer selon la revendication 4, dans laquelle les groupes polyoxyalkylène sont choisis dans le groupe constitué d'un polyoxyéthylène, d'un polyoxypropylène et de combinaisons de ceux-ci.

**6.** Composition aqueuse de soins personnels à rincer selon la revendication 5, dans laquelle l'éther de cellulose irréversiblement réticulé comprend un éther de cellulose de base et des réticulations ; dans laquelle les réticulations contiennent les groupes polyéther ; et dans laquelle l'éther de cellulose de base est choisi dans le groupe constitué d'hydroxyéthylméthylcellulose, hydroxypropylméthylcellulose, méthylhydroxyéthylhydroxypropylcellulose, éthylhydroxyéthylcellulose et combinaisons de ceux-ci.

**7.** Composition aqueuse de soins personnels à rincer selon la revendication 6, dans laquelle l'éther de cellulose de base est l'hydroxyéthylméthylcellulose.

**8.** Composition aqueuse de soins personnels à rincer selon la revendication 7, dans laquelle l'agent tensioactif de nettoyage dermatologiquement acceptable est choisi dans le groupe constitué d'alkyl polyglucosides, glycinates, bétaïnes, taurates, glutamates, sarcosinates, iséthionates, sulfoacétates, alaninates, amphoacétates, sulfates, sulfonates, succinates, alcanolamides gras et mélanges de ceux-ci.

**9.** Composition aqueuse de soins personnels à rincer selon la revendication 8, comprenant en outre un ingrédient facultatif choisi dans le groupe constitué d'un absorbant, d'un exhausteur esthétique, d'un acide alpha-hydroxylé, d'un agent antivieillissement, d'un antifongique, d'un agent antimicrobien, d'un antioxydant, d'un antiseptique, d'un agent antistatique, d'un agent bioactif, d'un agent de blanchiment, d'un agent chélateur, d'une matière colorante, d'un colorant, d'un émollient, d'un agent émulsifiant, d'un agent filmogène, d'un polymère fixateur, d'un agent moussant, d'un parfum, d'une particule dure, d'un humectant, d'un agent lubrifiant, d'un opacifiant, d'un agent perlant, d'un pénétrant, d'un agent d'ajustement du pH, d'un pigment, d'un extrait végétal, d'un conservateur, d'une protéine/d'un acide aminé, d'un modificateur de rhéologie, d'un sel, d'un modificateur sensoriel, d'un agent glissant, d'un savon, d'une particule molle, d'un additif pour écran solaire, d'un inhibiteur de lumière UV, d'une vitamine et de mélanges de ceux-ci.

**10.** Procédé de nettoyage d'au moins une peau et de cheveux de mammifère, comprenant :

(a) l'application d'une composition aqueuse de soins personnels à rincer selon la revendication 1 sur la peau ou les cheveux d'un mammifère ; et
(b) le rinçage de la composition aqueuse de soins personnels à rincer de la peau ou des cheveux avec une eau de rinçage.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8999391 B, Adamo
- US 2020155439 A

### Non-patent literature cited in the description


- **W. W. YAU** ; **J. J. KIRKLAND** ; **D. D. BLY**. Modern Size Exclusion Chromatography. Wiley-Interscience, 1979
- **J. P. SIBILIA**. A Guide to Materials Characterization and Chemical Analysis. VCH, 1988, 81-84
- **G. BARTELMUS** ; **R. KETTERER**. Zeitschriftfuer Analytische Chemie. Springer, 1977, vol. 286, 161-190